Europäisches Patentamt

European Patent Office

Office européen des brevets

(19)

(11) Publication number: **0 147 939**
**A2**

(12) **EUROPEAN PATENT APPLICATION**

(21) Application number: **84307914.6**

(22) Date of filing: **14.11.84**

(51) Int. Cl.⁴: **A 61 F 2/02**
**A 61 L 27/00, C 12 N 11/04**

(30) Priority: **15.11.83 US 551950**

(43) Date of publication of application:
**10.07.85 Bulletin 85/28**

(84) Designated Contracting States:
**AT BE CH DE FR GB IT LI LU NL SE**

(71) Applicant: **JOHNSON & JOHNSON**
**One Johnson & Johnson Plaza**
**New Brunswick, NJ 08933-7003(US)**

(72) Inventor: **Altman, Jean-Jacques**
**92 Rue Hauteville**
**F-75010 Paris(FR)**

(74) Representative: **Jones, Alan John et al,**
**CARPMAELS & RANSFORD 43 Bloomsbury Square**
**London, WC1A 2RA(GB)**

(54) **Implantable module for gylcemia regulation.**

(57) Implantable glycemic regulation module comprising semipermeable membrane encapsulating insulin-producing tissue from the group consisting of human insulinoma cells and human pancreatic islets.

_**Fig. 1**_

ACTUARIAL CURVE OF SURVIVAL OF GRAFTED AND CONTROL RATS

——— Normal rats(n=10) plus human insulin treated diabetic rats(n=6)

x—x—xUnprotected isogenic grafts (n=3)

•———•Long term plus short term successful protected insulinoma transplants(n=3+7) } n=21

————Unsuccessful protected insulinoma transplants (n=11)

————Protected isogenic grafts (n=6)

o—•—oNon-treated diabetic rats plus unprotected insulinoma transplants (n=8+1)

0147939

# IMPLANTABLE MODULE FOR GLYCEMIA REGULATION

## Field of the Invention

This invention relates to treatment of glycemia, and more particularly to implants which have the capacity over the long term to generate insulin, preferably in appropriate concentrations in response to changes in glucose concentration, and which use specific insulin-generating sources.

## Background of the Invention

Diabetes (more accurately known as Diabetes Mellitus) is one of the great unsolved medical problems of the world. For example, according to the National Institutes of Health, some ten and one-half million Americans have diabetes, although only approximately half of them have had the disease diagnosed. Further, the number of diabetics in the U.S. alone is increasing at a rate of five to six per cent per year. It is reasonable to assume that this also indicates the acuteness of the problem worldwide.

One reason for this growth is that diabetes affects many older people, whose numbers are on the rise. Another is that, because of better medical care, more diabetics are able to have children, some of whom grow up to become diabetics themselves through hereditary mechanisms. Finally, a growing school of thought holds that diabetes may be caused by viruses such as Coxsackie B4.

Of the patients with diagnosed diabetes, approximately five to ten per cent are absolutely dependent on insulin treatment. For the remaining portion, although survival does not depend on use of insulin, many do take insulin to relieve their symptoms. Most non-insulin dependent diabetics control their disease by using oral antidiabetic

**0147939**

drugs, by restricting their diets, or by exercising, partly to control their weight.

Diabetes alone is the fifth leading death cause in the United States, and coupled with its complications, diabetes is surpassed only by cardiovascular disease and cancer as a cause of death.  Among these complications are kidney disease, gangrene, cardiovascular disease, and damage to the blood vessels and nervous systems.  Among the serious but non-lethal complications of diabetes are cataracts, retinal damage, and even blindness.

Notwithstanding the prevalance of diabetes, and the tremendous progress made in recent years in understanding and treating it, much remains to be learned concerning the nature of diabetes and its treatment.  In fact, much remains to be learned about the operation of the pancreas and the hormones which it generates.

The body produces insulin in the pancreas, a large gland below and behind the stomach.  The pancreas contains two functionally different types of cells, including the acinar cells which make enzymes that promote digestion in the small intestine, and the islets of Langerhans (commonly known as the islets) which secrete several hormones, including insulin, into the blood stream. Each islet contains at least four types of cells, the most important of which are the alpha, beta, and delta cells.  Alpha cells secrete the hormone glucagon which increases the glucose level in the blood.  Beta cells secrete insulin, which decreases the blood's glucose level. Delta cells secrete the hormone somatostatin, which slows the secretion of both insulin and glucagon.

In a normal person, all three hormones work together to maintain the glucose level at about 80 to 100 milligrams per 100 milliliters of blood.  If a person eats a meal rich in carbohydrates, the islets release insulin to lower the level of glucose.  However, if too much insulin is released and the glucose level falls too low, glucagon is secreted to raise it to normal.

Following the discovery of insulin, the prevailing scientific view was that diabetes had a single cause-- failure of the pancreatic islets to produce enough insulin. Moreover, diabetes was believed to produce a single metabolic defect--the presence of excessive amounts of glucose in the blood.  Recent research makes it clear that diabetes is much more complex, in fact constituting a group of diseases with a variety of causes--genetic, environmental, immunological, and so forth--but with at least a single feature in common:  the body's inability to metabolize glucose normally.  An excellent overview of many of the current theories of the nature of these diseases, and some of the more recent approaches to treatment, is set forth in a Special Report in the March 2, 1981, Chemical and Engineering News, pp. 30-45, entitled "DIABETES--RAPID ADVANCES, LINGERING MYSTERIES", by H.J. Sanders, from which much of the foregoing was reproduced.

Prior Art Approaches

One recent popular approach to the treatment of diabetes has favored the development of external or implantable insulin pumps.  These pumps typically inject insulin continuously into the bloodstream through a needle or catheter, and often feature manual adjustments which allow the patient or treating physician to change the amount of insulin being injected.  Such pumps promise to address problems attendant to periodic bolus injections of insulin, in that the patient need not cope with resulting swings between high and low glucose levels. State of the art pumps are not, however, truly responsive to precise glucose levels on an instantaneous basis, and lack the capacity to adapt insulin flow precisely in response to real-time changes in blood glucose. Pumps also fail to afford the dynamic adaptation which occurs in the patient's body through complex interactions of the various pancreatic hormones.

Considerable effort also has been directed to the production of an implantable artificial hybrid pancreas. These schemes, which are exemplified by U.S. Patents 3,734,851 and 3,827,565 to Matsumura, and British Patent 1,479,002 to Spielberg, as well as numerous others, constitute a blood-islet cell insulin exchanger. Typically, cells are disposed in a housing through which blood is passed. In some, the blood carrier is a tubular ultrafiltration membrane, whereas in others the islet cells are layered or sandwiched between ultrafiltration membranes. In either case, the blood flows through the housing and pressure differentials across the ultrafiltration membrane cause the glucose-insulin exchange, while immunocytes and the like rejection mechanisms from the blood are blocked by the membrane and hence prevented from attacking the islet cells. See also the publication of W.L. Chick et al. entitled "ARTIFICIAL PANCREAS USING LIVING BETA CELLS: EFFECTS ON GLUCOSE HOMEOSTASIS IN DIABETIC RATS", Science 197:780--782 (1977) and numerous others. It is hypothesized that such "hybrid artificial pancreas" implants will work in a somewhat natural fashion, and hence provide safer, more efficacious regulation of glycemia than is provided by pumps. It appears, however, that while known hybrid artificial pancreas units do have short term beneficial tendencies in regulating glycemia, they are so subject to fibrosis and clotting as to be impractical other than for the shortest of time periods.  As a consequence, the membrances in popular consideration have short functional duration, severely limiting the utility of the implantation procedure.

It is a primary object of the present invention to provide methods and apparatus for regulation of human glycemia.  It is a further object to provide such methods and apparatus which respond to glucose changes in a substantially continuous, natural fashion, at least compatibly with the generation of the various hormonal agents produced by the normal pancreas in regulation of glycemia.  It is an associated object to provide implantable apparatus which is not readibly subject to immune/rejection mechanisms, and which avoids excessive fibrosis, which in the long run impedes the diffusion process in both directions (such device is thus less sensitive to blood glucose changes, and tends to build up insulin in the capsule).

Further, although the objects of the present invention are broad enough to embrace utilization of any suitable tissue, it is an object of the present invention to provide methods and apparatus appropriate to safe utilization of human insulinoma and islet cells.  Herein, the term "insulinoma" is utilized in its more or less conventional fashion, referring to a benign tumor of the islet cells, typically of the beta cells.  Insulinomas generally tend to be relatively disconnected from surrounding pancreatic tissue, to be heterogenous in terms of cell composition, and to secrete insulin at approximately the same (low) rate as a human islet cell.  Insulinoma cells do possess the vigor of most tumor cells, and hence, if safely enclosed and regulated, seem to be beneficial for diabetes treatment applications.  Insulinoma cells do not, however, have a highly consistent or predictable response characteristic to glucose challenge.

At the May 1981 International Symposium on Normal Glycemia for the Diabetic, Detroit, Michigan, J.J. Altman et al. in a talk entitled "ISLET AUTO TRANSPLANTATIONS IN PANCREATIC TUMORS" reported islet autografts as a preliminary step towards successful allografts in diabetic patients. In such autografts there were no rejection problems, but rejection is a major problem in allografts. It is, accordingly, an object of the present invention to provide methods and apparatus for glycemia regulation through safe and rejection-free use of insulinoma tissue.

A promising new approach to problems of islet transplantation and glycemia regulation has involved microencapsulation of individual pancreatic islet cells, which may then be implanted by injection. This work, which is reported by Lim and Sun and by Connaught Laboratories of Toronto, involves encapsulation of individual pancreatic islets in gel microdroplets (approximately 0.5 millimeters in diameter), in essence thereby enclosing the islet in a gelatinous membrane. These developments, however, are understood to be in the most tentative of development phases, and their technical detail as well as ultimate feasibility is believed an open question.

In a public disclosure by the inventor hereof to the July 8-10, 1981, ISAO meeting in Paris, France, in a presentation entitled "SUCCESSFUL PANCREATIC XENOGRAFTS USING SEMIPERMEABLE MEMBRANE", in the names of J.J. Altman, A. Manoux, P. Callard, D. Houlbert, N. Desplanque, F. Bruzzo and P. Galletti, there was disclosed the use of induced rodent insulinoma cells in semi-permeable membranes for control of glycemia. A paper subsequently was published based on the same data as disclosed in that oral presentation.

Continuing effort with respect to the data disclosed to the ISAO meeting has resulted specifically in effective glycemia regulation utilizing human insulinomas and human islets as the insulin-generating source in a membrane encapsulated pancreatic xenograft.

Summary of the Invention

The present invention is premised on the encapsulation and implantation of live human islet or insulinoma cells which produce insulin in relatively natural response to ambient changes of glucose levels. In accordance with the present invention, the cells are deposited in a capsule of membrane which permits natural diffusion of glucose into the capsule and thence to the cell, and diffusion of insulin out into the body. Nevertheless, the membrane capsule substantially blocks ingress of antibodies, immunocytes, and the like rejection mechanisms. The capsules are deposited into the liver, spleen or peritoneal cavity of the body, where they function in a nearly natural fashion in response to ambient glucose changes. Other sites may of course also be appropriate in accordance with the abilities of those of ordinary skill.

In one preferred embodiment, live human islet cells are deposited into an elongated tubular semipermeable membrane, which is sealed at both ends, and implanted directly into the liver, spleen, or peritoneal cavity of the patient. In such embodiment, the membrane material has a molecular weight cutoff approximately in the range of 50,000 daltons, and hence passes glucose and insulin but is a barrier to antibodies, immunocytes, and the like rejection mechanisms. In an alternative embodiment, the cells which are encapsulated are live insulinoma cells from a human donor.

A feature of the present invention is that the injected capsule, which typically is in the range of one centimeter or greater in length, and one-half to one and one-half millimeters inner diameter, senses glucose and emits insulin (and in some embodiments glucagon and somatostatin) through a mechanism of passive diffusion more similar to the natural pancreatic process than is the the flow created by pressure differentials across ultrafiltration membranes used in some of the prior art. Further, this relative concentration of cells, as compared with the microencapsulation, is believed beneficial, because the presence of diffusing insulin apparently has an inhibiting effect on further production of insulin, whereby overproduction by the insulinoma cells is essentially self-correcting. It is possible that encapsulating membranes associated with insulinoma cells enjoy more sustained viability, whereby the glucose insulin exchange and blockage of rejection mechanisms is extended relatively unabated and in relatively harmonious proportion.

Description of the Invention

The present invention is described in two prospective presentations. The substance of these presentations, set forth under sub-headings I and II below, comprise a complete description of the invention claimed herein. In the following description, reference is made by way of example only to the accompanying drawings, in which:

Figure 1 is an actuarial curve of survival of diabetic and control rats; and

Figure 2 shows some individual curves representative of the results shown in Figure 1.

I.  Macroencapsulated Human Insulinomas in
         Streptozotocin Diabetic Rats

or

# Long-Term Blood Glucose Normalization of Experimental Diabetes in Rats by Macroencapsulated Implants of Human Benign Insulinomas

In isogenic rats, transplanted islets from several healthy donors have been shown to correct most of the biochemical abnormalities associated with streptozotocin-induced diabetes, to prevent all or to revert most of the complications of the disease. While islet transplantation in main to correct insulin-dependent diabetes is safe, its achievement has been limited by the low yield of obtainable insulin-producing tissue and by the immune rejection problem. It has been suggested to use transplants protected by semi-permeable membranes to allow for allografts and xenografts. We report the effectiveness of immunospearation by a hollow fiber synthetic membrane in a pancreatic xenograft model using the transplantation of human benign insulinomas in streptozotocin-induced diabetic rats.

Material and Methods

One example of a fiber membrane useful in the present invention is a polyvinyl chloride acrylic copolymer capillary fiber with a 1.0 mm internal diameter and 110 $\mu$m wall thickness. Its molecular weight cut-off is approximately 50,000 daltons. This fiber, which is asymmetric (i.e., it exhibits a thin skin on the luminal wall surrounded by a honeycombed structure on the cuter side) is commercially available from Amicon Corporation (Lexington, Mass., U.S.A.) under the Amicon designation XM50. Segments of this material, 3 cm long, were filled with insulin-producing tissues and sealed at both ends with an occlusive glue. Other experiments with non-seeded fibers have been reported elsewhere. Endocrine tissue.

JJA-010

10

for 6 diabetic recipients was circa 1,000 freshly purified islets isolated from Wistar rats by the standard collagenase technique or, for 21 rats, benign human insulinoma tissues collected from 7 patients in 2 years. In all patients, the diagnosis of insulinoma was firmly established before surgery by inappropriately high levels of insulin or low levels of plasma blood glucose $\left(\dfrac{Ins}{PBG} \dfrac{\mu \, U/m}{mg/100 \, ml} > 1.37\right)$ and by localization of the tumor by selective angiography. Immediately after surgical removal the tumors were cut into square pieces of less than 500 $\mu$m using a tissue chopper (McIlwain from Mickle Laboratories). The tissues were kept at all times in Hanks medium at 4°C for a period of no more than 90 minutes between surgical removal and implantation of the encapsulated insulinoma.

Diabetes was induced in the recipient animals, inbred male Wistar rats weighing 200 gm, by a strictly intravenous injection of 45 mg of streptozotocin (Upjohn Co., Kalamazoo, Mich., USA). Rats were kept in individual metabolic cages and allowed to eat and drink ad libitum. Those who sustained more than 400 mg/100 ml BG levels for at least 15 days were used in transplant experiments. Protocol of transplantation is shown in Table 1.

11

Table 1

| Summary of Transplants | Diabetic Rats | Normal Rats |
|---|---|---|
| Protected Human Insulinoma Transplants | Peritoneal Cavity = 15<br>Liver = 4<br>Spleen = 2 | Peritoneal Cavity = 3 |
| Protected Isogenic Islet Transplants | Peritoneal Cavity = 6 | --- |
| Unprotected Human Insulinoma Transplants | Peritoneal Cavity = 1 | --- |
| Unprotected Isogenic Islet Transplants | Portal Vein = 8 | --- |

The fibers were implanted in each animal through a steel needle centrally located along the main axis of the spleen or the right lobe of the liver, or directly deposited into the peritoneal cavity. One fiber only was implanted in the liver and spleen sites. One to 3 fibers were implanted in the peritoneal cavity. Some non-implanted seeded fibers were fixed in Duboscq-Brazil's solution. Paraffin blocks were cut in 3-4 $\mu$m sections and stained with hematoxylin and eosin. Eight diabetic, untreated rats and 10 normal rats were observed in parallel with the experimental animals. Six diabetic rats were treated with 3 to 5 units daily of hemisynthetic human insulin (Novo) and 3 normal rats received an acute I.P. injection of the same insulin. Body weight of all rats was recorded every week and urinary output was controlled daily. Non-fasting plasma glucose (glucose oxydase) was measured twice weekly and plasma insulin levels once a week. In cases of rat islet transplantations, a guinea pig anti-serum against pig insulin was used because of its high affinity for rat insulin with rat insulin as a standard. In cases of insulinoma transplants, human insulin was assayed with insulin anti-serum and human insulin as standard. Rats were observed until their natural death, or death following the removal of the graft and not for more than a year for the successful grafts or the normal controls.

13

In vitro perifusion studies were performed in parallel with protected and unprotected fragments from three of the seven insulinomas before transplantation. In one case insulin release was studied with an insulinoma-seeded piece of hollow fiber membrane before and after 2 months of implantation. The samples were perifused in a Millipore chamber. Perifusion fluid was Krebs-Ringer bicarbonate solution pre-equilibrated with 5% $CO_2$ and 95% $O_2$ and entrapped in glass syringes. The liquid was perifused using a motor-driven pump (RT80 from Bioblock) at 0.25 ml/mn for 30 minutes at low glucose (2.25 mmol/l), followed by 60 minutes at 27.5 mmol/l (high glucose) and back to 2.25 for another course of 30 minutes. Human insulin release was measured every 3 minutes.

## Results

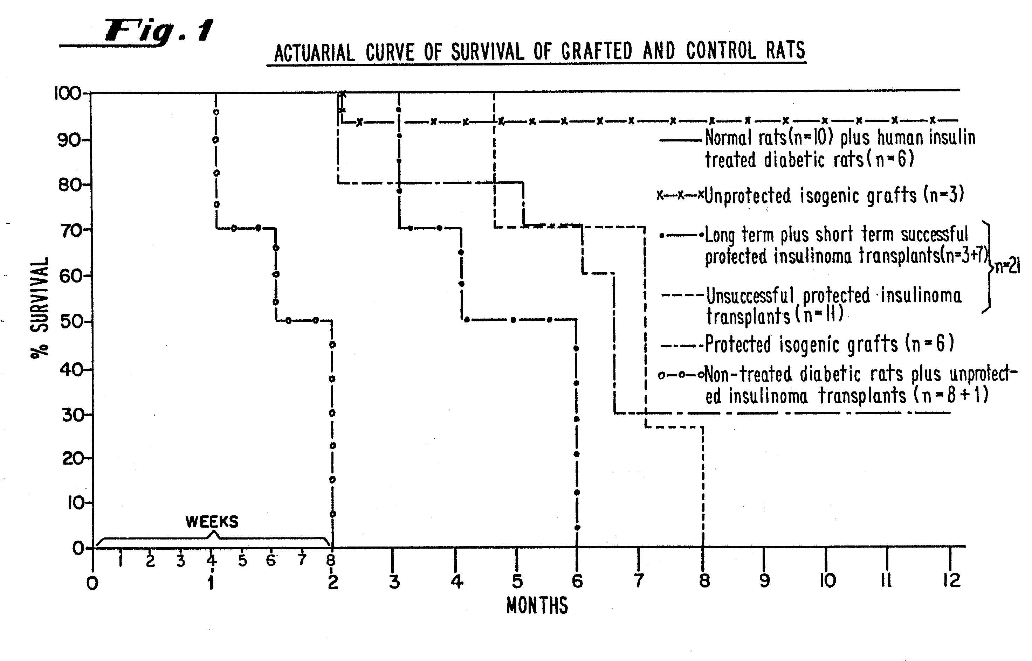

Weight gain was comparable to normal controls in successful transplants. Urinary output above 100 ml/day in diabetic controls or unsuccessful rats was under 5 ml/day in euglycemic rats with non-detectable glycosurea, compared to the 8 g/day in diabetic animals. Rate of plasma glucose and insulin normalization and degrees of duration of successful grafts are shown on Table 2. None of the animals receiving insulinoma transplants ever exhibited hypoglycemia. The actuarial curve of survival of all the experimental rats is shown in Figure 1.

Table 2

| Outcome of Transplants in Diabetic Rats | Unsuccessful | | Short-Term (1-4 Weeks) Successful (Normal Plasma Glucose and Insulin) | Long-Term (Over 4 weeks) Successful Ibidem) |
|---|---|---|---|---|
| | High Plasma Glucose, Low Insulin Level | But Prolonged Survival > 2 Months | | |
| Protected Insulinoma Transplants (n = 21) | 11 | 11 | 7 (Including 2 Rats With Surgical Removal of Grafts | 3 (> 6 Months) |
| Protected Isogenic Islet Transplants (n = 6) | 6 | 6 | --- | --- |
| Unprotected Insulinoma Transplants (n = 1) | 1 | --- | | --- |
| Unprotected Isogenic Islet Transplants (n = 8) | 1 | --- | --- | 7 |

JJA-010

- 15 -

In two of the permanently euglycemic rats grafted with protected insulinoma transplants, the hollow fiber membranes containing insulin tissues were surgically removed whereupon the animals became hyperglycemic and died within 15 days. Diabetic rats treated with human insulin were clinically comparable to normal rats with post-prandial blood glucose mildly elevated (160 ± 7 / 185 ± 12). The three normal rats with I.P. injection of 10 units of human insulin died within hours of hypoglycemia. In vivo study of post-prandial insulin levels is shown on Table 4. In vitro study of insulin release is shown on Table 5. Histology of non-implanted, seeded fibers reveals many insulin-secreting cells. as well as a complete filling of the hollow fiber membrane with no dead space available between the tissue and the wall of the fiber.

JJA-010

- 16 -

## Table 3

### In Vivo Study of Post-Prandial Insulin Levels

Normal Rats =   No Graft  = 48± 3 $\mu$U/ml

Xenograft = 32 ± 5 $\mu$U/ml


Diabetic Rats = No Graft  = 5 ± 0.5 $\mu$U/ml

Isograft  = 32 ± 3 $\mu$U/ml

Xenograft = Successful 52 ± 5

Failure    14 ± 2

Removed     9 ± 1

## Table 4

### In Vitro Study of Insulin Release

| Glucose | Insulinoma Before Implantation | Seeded Fibers | |
|---|---|---|---|
| | | Before | After Implantation |
| **Insulinoma A** | | | |
| 2.75 mmol/l | 210 $\pm$ 10 $\mu$U/ml | 150 $\pm$ 7 | 110 $\pm$ 9 |
| 27.5 mmol/l | 450 $\pm$ 17 $\mu$U/ml | 310 $\pm$ 22 | 285 $\pm$ 18 |
| 2.75 mmol/l | -- | -- | |
| **Insulinoma B** | | | |
| Low Glucose | 60 $\pm$ 4 | | |
| High Glucose | 68 $\pm$ 4 | -- | |
| Low Glucose | 63 $\pm$ 4 | | |
| **Insulinoma C** | | | |
| Low Glucose | 42 $\pm$ 4 | | |
| High Glucose | 39 $\pm$ 3 | -- | |
| Low Glucose | 35 $\pm$ 3 | | |

- 18 -

## Discussion

Several main lines have been followed in attempts to overcome the rejection phenomenon in non-isografted pancreatic islets. The most successful were the use of embryo pancreatic transplants such as the reversal of experimental diabetes in rats by chick embryo transplants obtained by Eloy; or the approach suggested by Lacy: prolonged survival of islet xenografts cultured in vitro for 7 days at 24°C before transplantation into rats which also received anti-lymphocytic serum; or the work of Lafferty: 7 days of pancreatic islet culture in high oxygen tension. First attempts of immuno-protection by semi-permeable membranes were hampered by the rapid development of fibrosis around the implant which, impairing the transfer of nutrient and metabolic messages to the encased cells, thus stops their adequate hormone delivery. Microencapsulation of single islets is successful, but the functional ability of the graft is limited in time. With the membrane-encapsulated pancreatic xenograph of this invention, tissue reaction and collagen deposition have been most of the time remarkably mild. Intestinal adhesions, abcess formation, as well as complete fibrotic encapsulation reported with the intraperitoneal implantation of diffusion chambers, were not observed. Similar good results were obtained by Archer using a

JJA-010

- 19 -

rodent islet-seeded fiber (XM100, Amicon Corporation) implanted in the peritoneal cavity of rats. We did not have successful grafts using protected isogenic islets in rats. In vivo insulinoma induces organic hypoglycemia because of the lack of negative feedback regulation: insulin level rises even when blood glucose declines. The first $\beta$ cell transplants in humans were performed with insulinomas: they failed in part because no immunosuppressive therapy was given. Insulinoma is not an overproducing hormone tumor: (a) it contains, most of the time, less insulin than the whole surrounding pancreatic gland but, contrary to normal islet cells, it can be recovered without loss of endocrine tissue in a few seconds; (b) our in vitro studies showed that some insulinomas responded to changes in the glucose concentration of the perifused medium. None of the animals receiving protected insulinoma transplants exhibited hypoglycemia. This may reflect a limited insulin production by a small amount of transplanted tissue, or the ability of the animal to compensate by continuous feeding. However, 24 hours of fasting in some of the successful grafts did not lead to hypoglycemia. This is not due to an inability of human insulin to lower blood glucose under a threshold level because an acute dose of human insulin induces a fatal hypoglycemia in normal rats, and adequate amount of human insulin normalizes blood glucose of diabetic rats. If a

0147939

protected insulinoma transplant can truly regulate BG levels in diabetic animals, the procurement of insulin-producing tissue could be partly resolved since each collected insulinoma could be used in several recipients and experimental insulinomas can be induced in rodents. Devices using living cells and semi-permeable membranes are studied under the generic name of bio-artificial organs. Many classifications have been proposed and an ideal bio-artificial pancreas could be described, including perfect kinetics of insulin and other islet hormone release and eternal life time of the membrane, without side effects. The virtual dead space between the cells and the membrane in our model, where insulin release under glucose stimulation is dependent on a diffusion process, may explain the blood glucose normalization of our diabetic rats. This protect:d insulinoma transplant model may not lead to perfect and permanent glucose regulation. However, this study estabishes the long-term effectiveness of immuno-separation by synthetic semi-permeable membranes. Other works are currently under investigation to optimize the membrane shape, surface characteristics and diffusive properties, as well as the search of other sources of insulin-producing tissues including pancreatic islets of various origins.

JJA-010

II.  Optimization of the Macroencapsulization Process
for Human Islets Transplanted in Diabetic Rats

A bio-artificial pancreas is a hybrid between a transplant and a prosthesis.  It combines the grafting of live tissues from a xenogenic donor with the use of synthetic polymer membranes to separate from the host the functional component of the implant.  To provide immunoseparation while allowing humoral exchanges between the device and the host, the selective permeability of the membrane material is chosen so as to permit the transfer of relatively small solute such as glucose and insulin while rejecting proteins, cells debris or living cells which could induce rejection.  We have previously reported long-term reversal of streptozotocin diabetes in rats by macroencapsulated implants of human benign insulinoma.  The aim of this work is a tentative optimization of the macroencapsulation process in rats together with the procurement of a new source of xenogenic insulin-producing tissue, human islets.

## Material and Methods

A.  Polymer Aspects of the Membrane

The hollow fiber semi-permeable membrane used in the present invention is shaped as a capillary.  The membrane used in our previous work was a hollow fiber of polyvinyl chloride acrylic copolymer. This asymmetric material exhibits an inner thin retentive skin surrounded by a larger honeycombed structure. Together with this fiber, 4 new fiber types have now been systematically studied. Their main characteristics, including specifically hydraulic permeability, and BSA and myoglobin rejection, were assessed and are indicated in Table 5. All of these fiber types have been successfully tested as the hollow fiber semi-permeable membrane in a pancreatic xenograft in accordance with the present invention.

For a workable hollow fiber semi-permeable membrane, it is essential that the membrane permits inward diffusion of glucose and other cell nutrients and outward diffusion of insulin, other endocrine hormones, and cell waste products while preventing inward penetration of immunocytes.

## Table 5

### Characteristics of Fibers

| Fiber Type | Molecular Cut-Off | Skin Structure | Dimensions | | Hydraulic Permeability | Rejection % | |
|---|---|---|---|---|---|---|---|
| | | | ID (mm) | Wall (μm) | | BSA | Myoglobin |
| Acrylic Copolymer (Amicon XM50) | 50,000 Daltons | Inner | 1.00 | 124 | 52.0 | 98 | 10 |
| Acrylic Copolymer | 50,000 Daltons | Double | 0.98 | 117 | 5 | 98 | 10 |
| Acrylic Copolymer | 50,000 Daltons | Outer | 1.16 | 51 | 0.8 | 96 | 45 |
| Poly-acrylate | 50,000 Daltons | Outer | 0.99 | 102 | 1.0 | 97 | 55 |
| Cellu-losic | 20,000 Daltons | No Skin | 0.81 | 26 | 0.4 | 97 | 68 |

- 24 -

B.  Tissue Harvesting - Aspects and Preparation

Four human pancreatic glands were obtained from four heart-beating cadavers (33, 35, 20 and 22 years old) in the same step as removal of their kidneys for grafts on hemodialyzed patients. · Just after the clamping of the splenic artery, the tail of the pancreas was resected and immediately deposited in 4°C cold Euro Collins where it remains until the end of islet isolation.  Weights of the pieces were, respectively, 35, 27, 30 and 38 grams.  The glands were immediately processed and islets were ready for any further use in less than 90 minutes, except in case 1 where the gland remained in cold medium 12 hours before use.  The four glands were first dissected with scissors in cubic pieces of about 3 mm length or smaller.  They were further minced with an industrial chopper (McIlwain from Mickle Laboratories) or with a home-made chopper.  On the McIlwain chopper, pieces are deposited on a plate which slices laterally completely in 20 seconds under an arm mounted with a razor blade, giving 50 strokes in 10 . seconds.  A 90° turn of the plate allowed to obtain square pieces, the size of which, 500 $\mu$m, is determined by a graduated screw positioned before the mincing.  One gram of tissue can be minced in about 1 minute. The home-made chopper is a motor-driven plunger terminated by a grid in contact with a cruciform steel blade. Pieces of pancreatic tissue are deposited in a small beaker into which the running plunger is introduced. Five grams of tissue are evenly chopped in about 2 minutes. Pieces obtained after any of the chopping are checked under a dissecting microscope (10 to 40 magnification): identified islets are hand-dissected with the help of an iridectomy scissor. About 200 partially purified islets can be recovered in an hour. Collagenase is not used in this purification process.

C. Endocrine Tissue Evaluation

1. Histology

At each step of isolation, whole gland, scissor dissection, industrial or home-made chopper, hand-dissection of islets, samples are taken and fixed in Duboscq-Brazil's or Bouin Holland or Karnovski fixative, respectively, for light and electron microscopy. For light microscopy the fragments were embedded in paraffin. Sections 3 to 4 microns were stained with hematoxylin eosin and trichrome of Masson. Argentic stain

JJA-010

- 26 -

according to Grimelius was used to show granules of secretion. Immunoperoxidase method was applied to insulin (K512 PAP kit from Dakopatts).

2. **In vitro Evaluation**

In vitro perifusions were conducted with randomly-selected purified microfragments of each of the four pancreas deposited on the nitex filter of a Millipore chamber. Krebs-Ringer bicarbonate buffer equilibrated with 5% $CO_2$ and 95% $O_2$ was perifused at .25 ml/mm for 60 minutes at 2.2 mmol/1 glucose, for 30 minutes at 22 mmol/1 glucose and for an additional 40 minutes back at low glucose. Samples for insulin determination were taken every 5 minutes (human insulin anti-serum and human insulin as standard).

D. **Fiber Handling and Preparation**

Fibers are fragile and can be easily crushed if care in handling is not used. They are stored at 4°C. They contain glycerine as a humectant to allow the pores to easily wet. Glycerine is washed from the fiber by soaking pieces in water for approximately an hour with gentle stirring. For cleaning, fibers are transferred 1 hour in 0.1 N HCl with ultrasonic agitation, followed by 3 rinses with sterile saline. Empty fibers are plugged by sequentially clipping about 3 mm of each end in the appropriate occlusive glue and immediately quenching the end in sterile water. Empty, sealed fibers, kept in sterile water, are readily used after their preparation. For islet-seeded fibers, one end only is glued and about 200 human islets are introduced into the fiber. The second end is plugged as previously described. The fiber is stored in cold Euro Collins for the shortest time possible before rat implantation.

E. Fiber Implantation

1. Empty Fibers

Pieces of fiber, 3 cm long, were implanted in the spleen, liver or peritoneal cavity of Wistar rats weighing approximately 200 g. For spleen implantation, a steel catheter was centrally located along the main axis of the organ and withdrawn while introducing the fiber. The same procedure was used for the upper lobe of the liver. Fibers were directly deposited into the peritoneal cavity. One fiber only was implanted in each rat. Fibers of 5 types (listed in Table 5) were implanted in the 3 sites of at least 5 rats for each of the following durations of time: 15 days, 1 month, 3 months, 6 months, and 12 months, except the

JJA-010

cellulosic fiber (no more than 6 months). Fibers were surgically removed with surrounding tissue and immediately processed as previously described for light and electron microscopy. A sample was randomly taken in the middle of each fiber and near one extremity.

2. Seeded Fibers

Diabetic Wistar rats (45 mg/kg of tail vein injection of streptozotocin, Kalamazoo) with sustained blood glucose of more than 400 mg/100 ml received peritoneal implants of 1 cm long. The first 3 pancreas provided implants for 3 rats each and the last 1 for 5. One to 3 fibers were grafted in 14 diabetic recipients, while 10 non-treated diabetic rats were observed in parallel as controls. Blood glucose level was checked twice weekly the first month, then weekly as insulin levels. All samples were non-fasting. Each grafted rat was kept in an individual metabolic cage to allow urinary output collection. Animals could drink and eat ad libitum. They were observed for periods of up to 6 months following implantation.

Results

Human Pancreas

Light microscopy revealed nice preservation of the 3 intact pancreas immediately processed. Surprisingly, the gland left 12 hours in cold medium showed still well-preserved endocrine cells. Alterations caused by scissors were mild or nil, while those induced by the home-made chopper were higher than those of the industrial chopper. PAP test for insulin was homogenous for the original pieces.

Electron microscopy findings were in accordance with light examination. In initial pieces, normal geography was preserved along with intracellular structures. With the home-made chopper, complete disruption of all organelles of endocrine cells was too frequently observed. Scissors induced only mild structural damage consisting primarily of dilatation, vesiculation and degranulation of endoplasmic reticulum. In between aspects were seen in pieces minced with the industrial chopper: mild alterations of intracellular organelles and their structural relationships.

Perifusions

In perifusion studies, the 4 batches of islets responded in the same range.

JJA-010

- 30 -

Capsule Interactions with Environment

A score from 0 to ++++ was attributed for each site and each duration of implantation. 0 means no reaction; +, 1 monocellular layer; ++++, layer of cells as thick as the wall of the fiber; ++ and +++ are intermediary thickness between + and ++++. Duration of implantation did not influence significantly the time reaction which was stable between 1 and 12 months. The chemistry of polymer did not influence either reaction. At 6 months, according to the various sites, the score was as follows, all polymers combined:

JJA-010

Percent of Fibers With Each Score at 6 Months

|  | 0 | + | ++ | +++ | ++++ |
|---|---|---|---|---|---|
| Peritoneal Cavity | 50% | 5% | 40% | 5% | --- |
| Liver | 20% | 10% | --- | 20% | 50% |
| Spleen | --- | 30% | 40% | 30% | --- |

JJA-010

- 32 -

Layers of reactive tissue consisted primarily of histo-cytes, granulocytes, small lymphocytes and fibroblasts with remarkably mild collagen deposition. Many intraperitoneal implants exhibited only a continuous meso-thelial lining. The polymers never exhibited any degradation of their structure even after a year. Granuloma and capillary proliferation were not observed. Fibers with an outer skin showed no cellular infiltration in the voids off their wall. Fibers with an inner skin were invaded by polymorphonuclear leucocytes and fibroblasts with minor collagen deposition at the interface. In all cases, cells were restrained from entering the lumen by the condensed inner or outer layer of synthetic polymers and the plugs.

Grafted Animals

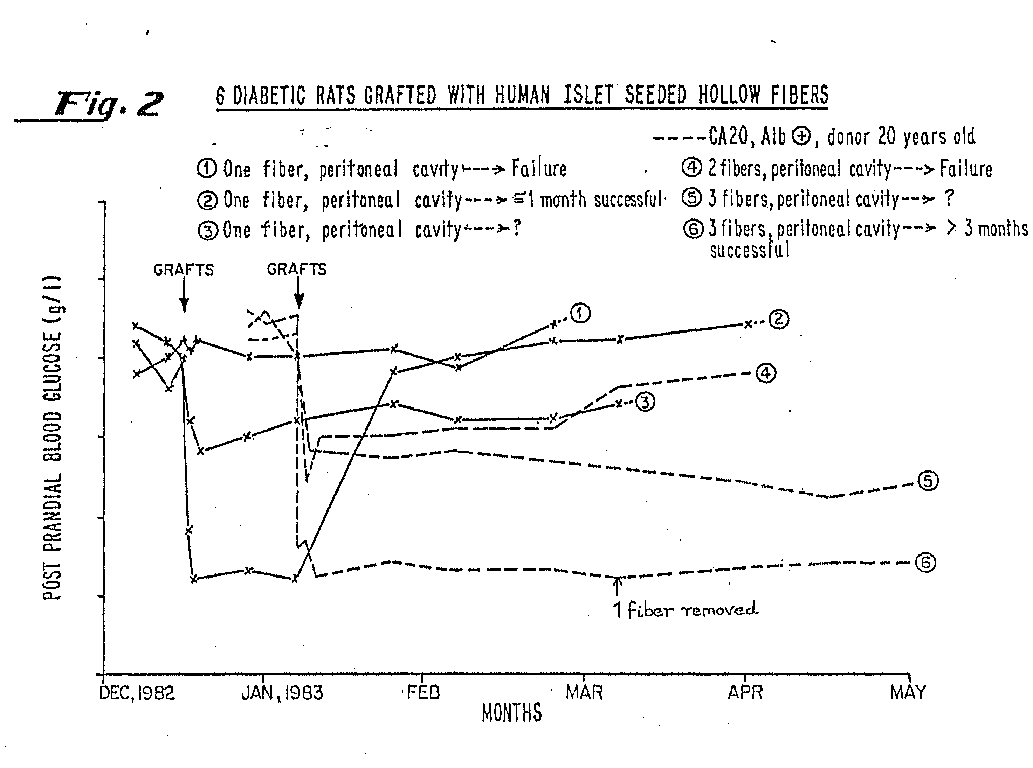

All 14 animals exhibited a prolonged survival compared to untreated controls. Post-prandial glucose levels were unchanged (1) in 5 animals (475 mg $\pm$ 52), significantly decreased (2) in 4 (228 $\pm$ 13) and fully normalized (3) in 5 (167 $\pm$ 10) for periods in excess of 3 months. Mean survival time in groups (1) and (2) was 20 weeks $\pm$ 4 / 5 $\pm$ 2 in the control group. Figure 2 shows some individual curves representative of the whole experiment. Insulin levels were in accordance with blood glucose normalization (not included). In 1 case, 1 fiber was removed from a euglycemic rat which kept normal blood glucose because 2 other fibers were left in place. Urinary output was normalized in successful rats (M = 6 ml/24 hours $\pm$ 0.5 / 137 $\pm$ 12).

Discussion

We have reported that semi-permeable, hollow fibers filled with benign human insulinoma tissue and implanted in rats could correct the hyperglycemia of streptozotocin-induced diabetes for several months. However, even if experimental insulinomas can be induced in rodents, tissue procurement may be a limiting factor in this model. On the other hand, insulinoma tissue could lack of biological feedback regulation of insulin reaction, inducing a risk of hypoglycemia. Selection of human islets donors with respect to age and improvements in the harvesting technique should allow a high yield of functional tissue and increase the effectiveness of the bio-artificial pancreas. Yield is still a significant problem in the field of islet graft. Most of the emphasis in islet isolation has been put in the late phase of the method, the enzymatic digestion. In fact, the first step, mechanical digestion, is of crucial importance. Differences observed between the two choppers were not due to inadequate fixation, anoxia, osmotic stress or autolytic digestion but to mechanical damage, because all the samples were in the same state of preservation

0147939

- 34 -

before chopping. An improved home-made chopper has been designed for this purpose. The absence of fibrous capsule formation is an important factor in the effectiveness of diffusional transport to and from macroencapsulated implants. We have never observed complications such as intestinal adhesions, abcess formation or fibrotic encapsulation which have been reported with the intraperitoneal implantation of other diffusion chambers. We find that it is difficult to infer from the in vitro data to actual results in vivo. We failed to demonstrate a clear influence of geometry (i.e., with or without skin, and/or inner, and/or outer skin, different diameters still in the same range or larger variations in wall thickness). Definitely more uniform results were seen in the peritoneal site. The main factors contributing to the success of this design are the control of the fibrous tissue reaction and the functional duality of grafted tissue. Comparison of histo-logical, in vitro and in vivo experiments could provide a model for clinically successful implants. While other approaches are currently under investigation to solve the immune rejection problem, our work demonstrates the long-term effectiveness of immuno-separation by semi-permeable membranes as well as the ability, to a certain degree, of normal isolated human islets to normalize blood glucose in experimental diabetes in rats.

0147939

The present invention will therefore be understood to encompass several aspects in various permutations, relating to the selection, composition, and configuration of the capsule, selection of the cell material and disposition therein, and the placement of the capsules into the body of the patient.

First, for example, the foregoing mentions the Amicon XM 50 membrane material as a preferred embodiment. This material, as well as others, is described in U.S. Patent 3,615,024, which gives considerable guidance to those of ordinary skill in the art with respect to the characteristics, preparation, and utilization of appropriate membrane materials. See also the article entitled "MEMBRANES" in Technology, Vol. 2 No. 2 March/ April 1982, pp. 16-29. Numerous alternative materials will become available to those of ordinary skill in the art, based upon the foregoing sources and the progress in the art. The threshold attributes of the material include the relatively free passage, through the mechanism of diffusion, of glucose and insulin, in both directions through the membrane. Immunologic rejection mechanisms, however, are to be blocked thereby. Depending whether a "skin" is to be formed on the inside and/or outside of the membrane tube, formation of cellular structure in integral fashion about the tube may or may not be promoted. This is believed acceptable in either case, depending of course upon the desires of the designer, provided the formation of tissue in and about the tubular membrane structure is of a conventionally cellular and vascular nature, avoiding fibrosis and clotting which not only would be detrimental to free flow of insulin and glucose, but furthermore which would be dangerous to the patient recipient of the implant.

Capsules may be sealed as simply as by utilization of epoxy or the like sealants, or by more elaborate polymerization techniques compatible with the membrane capsule.

As mentioned hereinbefore, the present invention is amenable to implantation of human biologically normal islet cells and islets, as well as insulinoma. Clearly, providing the auto regulation functions are adequately achieved and the cell viability is duly provided for, normal islets or islet cells seem to be a preferable alternative. Notwithstanding their benign nature, insulinoma cells provide considerable caution to those of ordinary skill in the art as an implantable material. Nevertheless, to the extent that hazard-free implantation is assured by the proper selection and sealing of the membrane capsules, the viability of the insulinoma cells may at least for some applications provide a preferable cell material selection.

In accordance with the present invention, no substantial limitations as to placement of the capsules are seen. In particular, one of the outstanding features of the principles of the present invention is that the membrane enclosed insulin producing cells need not be positioned for direct coupling of the insulin into the blood. For many applications, contact with blood will not even be desirable. In accordance with the passive diffusion scheme contemplated in accordance with the present invention, capsules have been successfully implanted in the livers and/or spleens of animals, and clearly are functionally acceptable deposited generally in the peritoneal cavity of the host. In fact, the latter implantation scheme may for most applications be desirable, in that the highly vascular nature of both the liver and the spleen, particularly in the human, presents a hazard to the injection of the implantable capsules directly into such organ. On the other hand, to the extent that such implants may be made safely, the vascular nature no doubt would promote the effective operation, and auto regulation of glycemia levels through implantation in accordance with the principles of the present invention.

Finally, the mode of disposition of the capsules into the host is not understood to be critical in accordance with the principles of the present invention. The size of the capsules may well lend themselves to direct injection through mechanism of a syringe or the like, but it may for some applications be desirable to implant them in different ways, for example through incision and surgical placement, or through intermediate placement mechanisms. In this regard, it is noteworthy that ultrasound techniques for guidance of aspiration biopsy needles into the body have undergone considerable recent investigation, and presently provide considerable sophistication and control for the surgeon. Clearly, such an approach could be utilized with great skill and advantage in accordance with the principles of the present invention, with the capsules being injected through the aspiration needle once the ultrasound imaging/location system indicates proper placement of the tip of that needle.

0147939

Claims

1. An implantable module for regulating human glycemia comprising a live insulin-producing source, from the group consisting of a plurality of human insulinoma cells and a plurality of human islets, said source being encapsulated in a hollow fiber membrane material, said membrane material being permeable to insulin and other endocrine hormones, cell waste products, and cell nutrients, and impermeable to immunocytes.

2. The module of claim 1, wherein said insulin source comprises live human insulinoma cells.

3. The module of claim 1, wherein said insulin source comprises human pancreatic islets.

4. The module of any one of claims 1 to 3 for use in regulating human glycemia.

5. A method of producing a module according to any one of claims 1 to 3, comprising encapsulating a live insulin-producing source, from the group consisting of a plurality of human insulinoma cells and a plurality of human islets, in a hollow fiber membrane material, said membrane material being permeable to insulin and other endocrine hormones, cell waste products, and cell nutrients, and impermeable to immunocytes.

**Fig.1** ACTUARIAL CURVE OF SURVIVAL OF GRAFTED AND CONTROL RATS

Legend:
- ——— Normal rats(n=10) plus human insulin treated diabetic rats(n=6)
- x—x—x Unprotected isogenic grafts (n=3)
- •——• Long term plus short term successful protected insulinoma transplants(n=3+7)
- - - - Unsuccessful protected insulinoma transplants (n=11)
- -·-·- Protected isogenic grafts (n=6)
- o—o—o Non-treated diabetic rats plus unprotected insulinoma transplants (n=8+1)

} n=21

% SURVIVAL (y-axis)
WEEKS / MONTHS (x-axis)

1 / 2

0147939

**Fig. 2** 6 DIABETIC RATS GRAFTED WITH HUMAN ISLET SEEDED HOLLOW FIBERS

----CA20, Alb ⊕, donor 20 years old

① One fiber, peritoneal cavity ---→ Failure
② One fiber, peritoneal cavity ---→ ≦1 month successful
③ One fiber, peritoneal cavity ---→ ?
④ 2 fibers, peritoneal cavity ---→ Failure
⑤ 3 fibers, peritoneal cavity --→ ?
⑥ 3 fibers, peritoneal cavity --→ > 3 months successful

POST PRANDIAL BLOOD GLUCOSE (g/l)

GRAFTS    GRAFTS

1 fiber removed

DEC,1982    JAN,1983    FEB    MAR    APR    MAY

MONTHS